(19) Europäisches Patentamt
European Patent Office
Office européen des brevets

(11) Publication number: **0 100 604**
**A1**

(12) # EUROPEAN PATENT APPLICATION

(21) Application number: 83303852.4

(22) Date of filing: 01.07.83

(51) Int. Cl.[3]: C 07 C 1/20
C 07 C 9/12, C 07 C 11/09
B 01 J 29/04
//B01J29/28

(30) Priority: 02.07.82 US 394795

(43) Date of publication of application:
15.02.84 Bulletin 84/7

(84) Designated Contracting States:
DE FR GB IT NL

(71) Applicant: SUMMIT GAS SYSTEMS PTE. LTD.
43 Jalan Buroh
Juron 2261(SG)

(72) Inventor: Anthony, Rayford G.
1102 Sul Ross Drive
Bryan texas 77801(US)

(72) Inventor: Thomas, Pullolickal Eapen
Pullolickal Thottakad P.O. 685 539
Kottayam District Kerala State(IN)

(74) Representative: Perry, Robert Edward et al,
GILL JENNINGS & EVERY 53-64 Chancery Lane
London WC2A 1HN(GB)

(54) **Process for converting methanol and/or dimethyl ether to iso-c4 compounds, and catalysts for use therein.**

(57) Methanol and/or dimethyl ether is converted to a hydrocarbon product rich in iso-$C_4$ compounds by contact with a catalyst comprising crystalline silica having molecular sieve properties or a crystalline solid with molecular sieve properties which has been impregnated with one or more of thorium oxide, zirconium oxide and titanium oxide. A novel catalyst comprises the thus-impregnated crystalline silica.

EP 0 100 604 A1

Croydon Printing Company Ltd

PROCESS FOR CONVERTING METHANOL AND/OR DIMETHYL ETHER TO ISO-$C_4$ COMPOUNDS, AND CATALYSTS FOR USE THEREIN

This invention relates to a process for converting methanol and/or dimethyl ether to a hydrocarbon product, particularly a hydrocarbon product rich in isobutane and isobutene, and to catalysts for use therein.

Isobutene or isobutylene and isobutane are important starting materials for the preparation of a variety of commercially important products, including maleic anhydride-isobutylene copolymer, 2,5-dimethylhexadiene (which is a key intermediate in the manufacture of pyrethrin insecticides), butyl rubber, polybutenes, methyl methacrylate,and alkylate and methyl t-butyl ether (which are blending components for high octane gasoline).

At present, isobutylene is obtained generally from the cracking of liquid petroleum or from naturally-occurring field butanes. However, the demand for isobutylene far exceeds the supply, and this demand is increasing.

The synthesis of branched chain hydrocarbons from synthesis of water gas, i.e. a mixture of carbon monoxide and hydrogen, has been investigated at the Kaiser Wilhelm Institute for Coal Research, Germany; according to a translation of a report by Pichler and Ziesecke, Bureau of Mines Bulletin, 488, United States Government Printing Office, Washington, D.C. 1950, water gas has been converted under high pressure and elevated temperature to a hydrocarbon product containing isobutylene using a variety of catalysts, including catalysts composed principally of an oxide of thorium, aluminium, tungsten, chromium, titanium, zirconium, uranium, zinc, manganese or cerium and mixtures of thorium oxide with alumina, zinc oxide, chromium oxide, iron and copper.

While the work of Pichler *et al*. shows that water gas can be converted into a hydrocarbon product containing

iso-$C_4$ hydrocarbons, the Pichler *et al*. process has serious shortcomings which have prevented its commercial use. In particular, the conversion is carried out at very high pressure, i.e. from 30 to 1000 atm. The higher the pressure, the better the conversion, but high pressure equipment is very expensive. Further, thorium oxide is also costly and the concentration of thorium oxide in the Pichler *et al*. catalyst is high, i.e. about 71-100% by weight.

More recently, crystalline aluminosilicate zeolites or molecular sieves have been disclosed and shown to be useful for catalysing a variety of reactions. For example, US-A-3,036,134 discloses that alcohols may be converted to ethers using a crystalline aluminosilicate of the formula:

$$M_{2/n}O:Al_2O_3:XSiO_2:YH_2O$$

wherein M is a metal and n its valency, X varies from about 1.35 to 3 and the average value for Y is between 4 and 8.

US-A-3,529,033 discloses the dehydration of a normal alcohol to an olefin having a corresponding structure, using such a synthetic zeolite or molecular sieve.

US-A-4,079,096 discloses that a catalyst of crystalline aluminosilicate zeolite is useful for converting methanol and/or dimethyl ether to a hydrocarbon product rich in ethylene and propylene. The same conversion is disclosed in US-A-4,062,905 and DE-C-2,827,385 and 2,755,229. Reactions of polar organic compounds using zeolite catalysts are disclosed in US-A-3,728,408; the production of aromatic compounds from liquid hydrocarbons using a crystalline aluminosilicate catalyst is disclosed in US-A-3,756,942 and 3,760,024; and the aromatisation of alcohols, ethers and carbonyl-containing compounds using a crystalline aluminosilicate zeolite is

disclosed in US-A-3,894,104. The use of a crystalline aluminosilicate zeolite catalyst to convert lower aliphatic alcohols, carbonyls and ethers to a hydrocarbon product rich in compounds containing 5 or more carbon atoms is disclosed in US-A-3,894,103, while the conversion of a compound of formula $CH_3X$, wherein X is a halogen atom or hydroxyl, alkoxy, alkylthio, amino, alkylamino, dialkylamino or cyano group, to an aromatic compound is disclosed in US-A-3,894,105.

US-A-4,061,724 discloses a crystalline silica composition of uniform pore diameter, which exhibits molecular sieve and hydrophobic/organophilic, but not ion-exchange, properties. This silica composition, which is referred to as silicalite, is substantially free of alumina except for incidental impurities. The crystals are orthorhombic. Silicalite has also been characterised as a silica polymorph which, after calcination in air at 600°C for 1 hour, has a mean refractive index of 1.39 and a specific gravity at 25°C of 1.70. Owing to its uniform pore structure, silicalite is disclosed as being useful for separating p-xylene from o-xylene. Silicalite is said to be useful for selectively absorbing organic materials from water as a result of its hydrophobic/organophilic properties.

According to the present invention, a process for preparing a hydrocarbon product rich in iso-$C_4$ compounds comprises contacting methanol and/or dimethyl ether with a catalyst comprising crystalline silica which has a substantially uniform pore structure and exhibits molecular sieve properties, at 300 to 550°C.

According to a further aspect of the invention, such a hydrocarbon product is obtained by a process which comprises contacting methanol and/or dimethyl ether, at 300 to 550°C, with a catalyst having molecular sieve properties which has been impregnated with one or more of

the oxides of thorium,zirconium and titanium.

The catalyst should not be amorphous silica. It may be a crystalline silica which exhibits molecular sieve properties and is substantially free of alumina, such as the silicalite disclosed in US-A-4,061,724. The catalyst preferably has a uniform pore size of 5 to 10, more preferably 5 to 8, and most preferably 5.2 to 6.5, Å.

If an impregnated catalyst is used, it comprises silica and is preferably a crystalline silica or crystalline aluminosilicate of uniform pore diameter (preferably of the values given above), e.g. silicalite impregnated with thorium oxide, zirconium oxide and/or titanium oxide. A catalyst which has been impregnated with thorium oxide is particularly preferred. While impregnated catalysts containing up to 50 parts by weight of oxide may be used, such catalysts preferably contain from 0.5 to 30, more preferably 2 to 15, parts by weight of the oxide.

The oxide-containing catalyst may be prepared by any convenient procedure. For example, the crystalline molecular sieve composition may be combined with an aqueous solution of a water-soluble salt of the desired metal oxide. After the resultant slurry is dried, the catalytic composition is packed in a reactor and calcined at a temperature which does not destroy the crystal structure of the catalyst, e.g. at 340 to 600°C. Thereafter, the catalyst may be cooled and maintained in an inert atmosphere such as nitrogen.

To produce a hydrocarbon product rich in iso-$C_4$ compounds, in accordance with the invention, methanol or dimethyl ether, or a mixture thereof, is fed to a reactor, into contact with the catalyst. Water may also be fed to the reactor with the methanol and/or dimethyl ether, e.g. in an amount of up to about 80, and preferably up to 70, % by weight of the feed. The temperature of

the reactor during the conversion is preferably 310 to 550, more preferably 330 to 455, and most preferably 370 to 430, $^{o}C$.

The conversion of methanol and/or dimethyl ether may be carried out at a pressure of $10^4$ to $2 \times 10^6$, more preferably $10^5$ to $10^6$, and most preferably $10^5$ to $4 \times 10^5$, Pa. However, while the conversion may be carried out at elevated or reduced pressure, it is a particularly advantageous feature of the process of the invention that the conversion may be carried out at ordinary atmospheric pressure, so that the expense of high pressure equipment may be avoided.

Examples of suitable feeds are methanol, a mixture of methanol and water, dimethyl ether, a mixture of dimethyl ether and methanol, or a mixture of methanol, dimethyl ether and water. Preferred feeds include methanol alone, a 90:10 to 30:70, more preferably 60:40 to 30:70, w/w mixture of methanol and water, and an equilibrium mixture of methanol, dimethyl ether and water. The equilibrium mixture is that obtained when the three components establish an equilibrium relative to the reaction:

$$2\ CH_3OH \leftrightarrow (CH_3)_2O + H_2O$$

at 250 to 450$^{o}C$. Preferably, the mixture is obtained by passing methanol over any dehydration catalyst known in the art, one of which is γ-alumina, at 250 to 450$^{o}C$. The equilibrium composition is dependent upon the initial feed composition and the reaction temperature. Methanol, dimethyl ether and water, methanol and water, or dimethyl ether and water can be converted to an equilibrium mixture at 250 to 450$^{o}C$ and used as the feed. Most economically, crude methanol, containing about 80 to 90% of methanol, with the remainder, i.e. 10-20%, water, with minor impurities, is used.

The conversion is desirably carried out at a

space velocity of 0.1 to 20, preferably 0.5 to 10, and most preferably 0.7 to 5, WHSV. Ethylene and/or propylene can be recycled to increase the yield of iso-$C_4$ compounds, if desired.

In a particular embodiment of the invention, methanol may be converted to a hydrocarbon product rich in iso-$C_4$ compounds in two or three stages. In such a process, a dehydrating catalyst such as γ-alumina is packed near the entrance of a reactor and the iso-$C_4$-producing catalyst, i.e. a silicalite, silicalite-oxide, or other molecular sieve-oxide catalyst, is packed below the alumina. Methanol is then fed to the reactor, which is converted by the γ-alumina in the first stage of the reaction to an equilibrium mixture of dimethyl ether, water and methanol. In the second stage, the equilibrium mixture is converted to the hydrocarbon product. A third stage can be added, to dehydrogenate isobutane in the hydrocarbon product to the more preferred isobutene.

All types of known catalytic reactors, including fluidised bed, recycle,moving bed, ebulating bed and tubular fixed-bed reactors, can be used in conducting the process of the invention.

The catalyst may include, if desired, an inert carrier or γ-alumina.

By means of the process of the invention, iso-$C_4$ compounds are obtained from methanol, dimethyl ether, a mixture thereof, or a mixture of any of the foregoing with water, in an amount which can be at least 10, preferably at least 20, more preferably at least 30, and often up to 50 (or more), % by weight of the gaseous products. An advantageous feature of the invention is that the ratio of isobutylene to isobutane in the product may be maintained in the range of 0.6 to 1:1 by feeding water with methanol or an equilibrium mixture of water, dimethyl ether and methanol to the reactor. Another

feature of this process is that a high content of iso-$C_4$ compounds in the gaseous hydrocarbon product can be maintained with the concomitant production of a limited amount of aromatic liquid product or of $C_2$ and $C_3$ hydrocarbons with a high olefin content. The production of liquid aromatics is reduced by cofeeding water with methanol into contact with the catalyst, and by increasing the flow rate.

The following Examples illustrate the invention. For each Example, gaseous product distribution was observed, and the results are tabulated, together with conversion parameters, after the Examples.

Example 1

Silicalite S-115 from Union Carbide was calcined at 468°C for three hours with an air flow rate of 60 $cm^3$/min. The calcined catalyst, herein designated A-1 (90 $cm^3$, 44 g), was placed in a tubular reactor, 19 mm in diameter and approximately 0.91 m long, surrounded by an electrical heater and fitted with a thermocouple.

Methanol was fed into the reactor packed with catalyst at a rate of 80 $cm^3$/hr (WHCV - 1.45 $hr^{-1}$) under a pressure of $5.5 \times 10^5$ Pa. The initial reactor temperature of 396°C rose to 424°C when methanol was introduced. Methanol conversion was 100%; 23.6 wt % of the ($CH_2$) group of methanol was converted to organic liquid, i.e. aromatic hydrocarbons.

Example 2

The catalyst used in Example 1 was regenerated by heating with air at a temperature of 482°C, at an air flow rate of 60 $cm^3$/min. After cooling the catalyst to 355°C with $N_2$, methanol was fed into the reactor (WHSV = 1.3 $hr^{-1}$). The temperature in the reactor rose to 430°C.

The reaction was continued for 85 min. to yield a total of 54.3 g of liquid product, including water and 8.24 g of liquid hydrocarbons. The methanol conversion

was 100%; 23.2% of ($CH_2$) was converted to aromatic hydrocarbons which were principally alkylated benzenes.

Example 3

The catalyst from Example 2 was maintained at 371°C in a flow of $N_2$ (60 $cm^3$/min) overnight and then cooled to 349°C.

When methanol was fed to the reactor the temperature rose to 432°C; WHSV = 1.63 $hr^{-1}$.

After 43 min., the liquid product collected

| | |
|---|---|
| including $H_2O$ was: | 58.70 g |
| liquid product excluding $H_2O$ | 15.35 g |
| $CH_3OH$ conversion | 100 % |
| DME (dimethyl ether) in product | 0 |
| % ($CH_2$) converted to organic liquid | 42.9 |

Example 4

The catalyst used in a preceding Example was kept in a current of $N_2$ (60 $cm^3$/min) overnight at 331°C. Methanol was fed into contact with the catalyst in the reactor (WHSV = 1.7 $hr^{-1}$). The following is the temperature profile during the conversion:

| Time (min.) | Temp. (°C) |
|---|---|
| 0 | 331 |
| 7 | 371 |
| 13 | 378 |
| 21 | 382 |
| 29 | 379 |

| | |
|---|---|
| Conversion of $CH_3OH$ | 100% |
| DME | 0% |

Example 5

The catalyst from a previous Example was cooled to 300°C in a flow of $N_2$ (60 $cm^3$/hr) for 5 hours. Methanol was then fed to the reactor packed with the catalyst; WHSV = 1.7 $hr^{-1}$.

The following is the temperature profile during the conversion:

| Time (min.) | Temp. ($^{o}C$) |
|---|---|
| 0 | 300 |
| 2 | 308 |
| 13 | 317 |
| 20 | 322 |
| 24 | 331 |
| 26 | 337 |
| 27 | 339 |
| 28 | 341 |
| 29 | 343 |
| 30 | 345 |
| 33 | 349 |
| 38 | 353 |
| 42 | 358 |
| 48 | 361 |
| 56 - 75 | 364 |

| | |
|---|---|
| Total weight of liquid product | 2.8 g |
| % ($CH_2$) conversion to organic liquid | 7 |

Example 6

90 ml (48.2 g) of silicalite (S-115) from Union Carbide were heated for 48 hours under vacuum. The thus-treated silicalite was slowly added to 100 $cm^3$ of 0.45 M $Th(NO_3)_4$ solution and mixed. After a time sufficient to ensure adequate impregnation of the silicalite, excess $Th(NO_3)_4$ solution was removed by filtration and the wet $Th(NO_3)_4$-impregnated silicalite was dried over a water bath and then heated to 180°C under vacuum for 72 hours. The catalyst was cooled, packed in the reactor, and heated to 471°C for 24 hours under a current of $N_2$ to convert $Th(NO_3)_4$ to $ThO_2$. $NO_2$ fumes were observed at the outlet of the reactor. The catalyst charged to the reactor weighed 40.1 g and occupied a volume of 65 $cm^3$. This is catalyst B-1. The maximum possible $ThO_2$ content would be 23% by weight.

The catalyst was cooled in $N_2$ to a temperature

of 313°C. Methanol was fed to the reactor into contact with the catalyst. The temperature profile of the reaction was as follows:

| Time (min.) | | Temp. (°C) |
|---|---|---|
| 0 | | 313 |
| 3 | | 343 |
| 7 | | 377 |
| 8 | | 440 |
| 13 | | 445 |
| 16 | (external heating stopped) | 452 |
| 23 | | 453 |
| 30 | | 450 |
| 37 | | 447 |
| 70 | (external heat started) | 425 |
| 80 | (external heat stopped) | 431 |
| 86 | | 442 |
| 100 | | 438 |
| 108 | | 432 |
| 118 | | 425 |
| 122 | | 421 |
| 130 | | 415 |
| 132 | | 414 |
| 136 | | 410 |
| 140 | | 408 |
| 143 | | 405 |
| 172 | | 383 |
| 187 | | 368 |
| 197 | | 357 |
| 200 | | 332 |

| | |
|---|---|
| Methanol conversion | 100% |
| DME in product | 0 |
| % $(CH_2)$ converted to organic liquid | 52.6 |

WHSV = 1.27 $hr^{-1}$

Example 7

A catalyst prepared as in Example 6 was used except that the initial temperature in the reactor was 342°C. A 1:1 v/v mixture of water and methanol was fed into the reactor. The temperature profile was as follows:

| Time (min.) | | Temp. (°C) |
|---|---|---|
| 0 | | 342 |
| 4 | | 363 |
| 5 | | 367 |
| 10 | | 375 |
| 11 | (heating discontinued) | 386 |
| 12 | | 391 |
| 14 | | 391 |
| 20 | | 388 |
| 26 | | 388 |
| 32 | | 393 |

| | |
|---|---|
| % conversion of $CH_3OH$ | 100 |
| % DME | 0 |
| % $(CH_2)$ converted to organic liquid | 22.7 |

WHSV = 1.27 $hr^{-1}$ (based on total feed)

WHSVM = 0.54 $hr^{-1}$ (based on methanol feed only)

Example 8

The same catalyst and methanol-water feed was used as in Example 7 except the initial temperature was 393°C. The temperature profile was as follows:

| Time (min.) | Temp. (°C) |
|---|---|
| 0 | 393 |
| 4 | 416 |
| 9 | 421 |
| 28 | 417 |
| 32 | 423 |
| 33 | 423 |
| 35 | 426 |

| | |
|---|---|
| 41 | 429 |
| 42 | 429 |
| 45 | 434 |
| 46 | 433 |
| 53 | 432 |
| 56 | 432 |
| 69 | 433 |
| 73 | 433 |

| | |
|---|---|
| % Methanol conversion | 100 |
| % DME in product | 0 |
| % $(CH_2)$ converted to organic liquid (aromatics) | 10.4 |

WHSV = 2.4 $hr^{-1}$ (based on total feed)

WHSVM = 1.0 $hr^{-1}$ (based on methanol feed)

The substantial increase in the production of $C_2$ and $C_3$ hydrocarbons, especially $C_2H_4$ and $C_3H_6$, and the decreased production of aromatics, in Examples 7 and 8 are due to the addition of water to the feed.

Example 9

A catalyst prepared as in Example 6 was maintained at a temperature of 329°C in a flow of $N_2$. Methanol was then fed to the reactor. The temperature rose to 458°C and then remained steady; samples were collected at 454°C.

| | | |
|---|---|---|
| Liquid products: | $H_2O$ | 58.9 g |
| | organic layer | 14.6 g |
| % methanol conversion | | 100 |
| % $(CH_2)$ converted to organic liquid | | 45 |
| DME in product | | 0 |
| WHSV | | 1.48 $hr^{-1}$ |

Example 10

A catalyst prepared as in Example 6 was maintained at a temperature of 378°C in a current of $N_2$. Air was passed through the catalyst for 1 hour and then the catalyst was cooled to 379°C. Methanol was fed to the reactor and heating was then discontinued.

| | |
|---|---|
| % $CH_3OH$ conversion | 100 |
| DME in product | 0 |
| % $(CH_2)$ converted to organic liquid | 42.1 |
| WHSV | 1.44 $hr^{-1}$ |

Example 11

Silicalite (23.98 g, 50 $cm^3$) and 18 $cm^3$ of a 0.45 M solution of $Th(NO_3)_4$ were mixed to form a thick paste which was dried to a powder (26.29 g) by heating over a water bath and then in an oven at 160°C at atmospheric pressure. The resultant catalyst, B-2, contained 8.4% by weight ThO.

Fresh catalyst prepared as above was packed in the reactor and heated overnight in a current of $N_2$ at 343°C. Methanol was then fed to the reactor at a flow rate that was varied during the run.

| | |
|---|---|
| Total $CH_3OH$ fed | 51.5 $cm^3$, 40.74 g |
| Total time of reaction | 90 min. |
| Wt. of liquid product | |
| Organic layer | 9 g |
| $H_2O$ | 22.72 g |
| % $(CH_2)$ converted to organic liquid | 50.5 |
| WHSV | 0.63 $hr^{-1}$ for 11 min. |

and then increased to 1.1 for remaining time

Example 12

Silicalite (60 g) was mixed with thorium nitrate solution (48 $cm^3$ diluted to 100 $cm^3$) to form a slurry. This was mixed with slight excess of $Na_2CO_3$ solution

(110 $cm^3$ of 0.425 M solution). Boiling $Na_2CO_3$ solution was added to the boiling $Th(NO_3)_4$ all at once (copious frothing was observed) while stirring vigorously. After thorough mixing, the solution was vacuum-filtered through Whatman (registered Trade Mark) No. 1 filter paper. The composition was then washed with 500 ml of deionised water and finally was dried over a water bath, to obtain 60.24 g of catalyst. One half of this (30.12 g) was slurried again with 10 $cm^3$ of $K_2CO_3$ solution containing 0.0143 g of $K_2CO_3$, dried again over a water bath, and calcined at 343°C in a muffle furnace, in a current of air (100 $cm^3$/min.) overnight; % ThO = 8.9, %$K_2CO_3$ = 0.05. This catalyst, numbered B-3, was packed in the reactor.

The catalyst was heated to 354°C in a current of $N_2$ (60 $cm^3$/min.) and then methanol was fed to the reactor. The temperature rose to 391°C and remained substantially constant for the length of the run.

| | |
|---|---|
| Time of run | 108 min. |
| % Conversion of ($CH_2$) to organic liquid | 42 |
| WHSV | 0.69 |

Example 13

Catalyst B-3 was used. A mixture of methanol and water (MeOH: $H_2O$, 9:1 by volume) was fed to the reactor.

WHSV 0.74 $hr^{-1}$ (based on total feed)
0.65 $hr^{-1}$ (based on $CH_3OH$ feed)

| | |
|---|---|
| % Conversion of $CH_3OH$ | 100 |
| % DME in product | 0 |
| % ($CH_2$) converted to organic liquid | 47.2 |

Example 14

Silicalite (90 $cm^3$, 48 g) was combined with 25 $cm^3$ of $Th(NO_3)_4$ solution which had been diluted to 100 $cm^3$ with water. Phosphoric acid prepared by dissolving 28.5 g $P_2O_5$ in 108 $cm^3$ of $H_2O$, and then by diluting

15 $cm^3$ of this solution to 100 $cm^3$, was added to the silicalite - $Th(NO_3)_4$ slurry in small quantities with constant stirring until no precipitation was noticed in the supernatant solution. Then a slight excess of the acid was added (in all about 80 $cm^3$). The solids in the beaker were separated by centrifuging, and washed three times using about 150 $cm^3$ of deionised water. The washings were also separated by centrifuging. Thorium phosphate content was estimated to be 6.6%.

The wet silicalite - thorium phosphate solid was dried over a boiling water bath and afterwards under vacuum at 140°C. 60 $cm^3$ (40 g) of this catalyst, numbered B-4, were packed in the reactor and calcined at 538°C. After cooling the catalyst to 371°C in a current of $N_2$, methanol was fed to the reactor for 69 minutes.

| | |
|---|---|
| % $(CH_2)$ converted to organic liquid | 52 |
| % DME in product | 0 |
| WHSV | 0.6 $hr^{-1}$ |

Example 15

A catalyst was prepared from silicalite in an amount of 25 g (51 $cm^3$) and 4 g of $Zr(NO_3)_2$. The zirconium nitrate was dissolved in 30 $cm^3$ of deionised water, and silicalite was added to form a slurry. After thorough mixing, the slurry was dried over a boiling water bath to dryness and then heated in the oven overnight at 140°C; the catalyst contained about 8.0% ZrO.

This catalyst, numbered C-1, was packed in the reactor and calcined overnight at 541°C in a flow of air; $NO_2$ fumes were observed at the outlet of the reactor; 54 $cm^3$ of catalyst were packed in the reactor. Methanol was fed to the reactor, which had been cooled to 379°C in a current of $N_2$.

| | |
|---|---|
| % Conversion of methanol | 100 |
| % DME in product | 0 |
| % $(CH_2)$ conversion to organic liquid | 60 |
| WHSV | 0.92 $hr^{-1}$ |

Example 16

The catalyst used in Example 15 was regenerated in a current of air (60 $cm^3$/min.) at 515°C and then cooled in a current of $N_2$ to 353°C. A methanol-water mixture (1:1 v/v) was fed to the reactor.

| | |
|---|---|
| Weight of liquid product | |
| organic liquid | 16.46 g |
| $H_2O$ | 32.17 g |
| Theoretical yield of $H_2O$ | 34.4 g |
| Theoretical yield of $(CH_2)$ | 27.5 g |
| % $(CH_2)$ conversion of organic liquid | 26.9 |
| % Conversion of methanol | 100 |
| % DME in gaseous product | 0 |

WHSV 2.36 (based on total feed)

WHSVM 1.05 $hr^{-1}$ (based on methanol)

The increase in the proportion of $C_2$ and $C_3$ hydrocarbons, especially $C_2H_4$ and $C_3H_6$, and the decrease in the proportion of aromatics in the product of Example 16, compared to the composition of the product of Example 15, are due largely to the presence of water in the feed.

Example 17

A few ml of $TiCl_4$ were poured directly into 75 $cm^3$ of water. The $TiCl_4$ hydrolysed, forming a white precipitate, probably of titanium oxychloride. On standing, the precipitate dissolved. The clear solution was then treated with excess $NH_4OH$. A thick white precipitate of the hydroxide which formed was filtered and washed repeatedly with deionised water until free of

Cl, and was then dissolved in a minimum amount of 1:4 dilute $HNO_3$.

30 $cm^3$ of the resultant titanium nitrate solution were diluted with 30 $cm^3$ of deionised water, and 32 g (60 $cm^3$) of silicalite were added; this mixture was heated to dryness over a water bath and then to 160°C for 4 hours. $NO_2$ fumes were observed during heating.

A silicalite - $TiO_2$ catalyst containing 7% by weight of $TiO_2$ was obtained, which was packed in a reactor and calcined at 531°C in air overnight and then cooled in a current of $N_2$ to 364°C. This catalyst is numbered D-1. Methanol was fed to the reactor.

| | |
|---|---|
| % $CH_3OH$ conversion | 100 |
| % DME | 0 |
| % $(CH_2)$ conversion to organic liquid | 48.7 |
| WHSV | 0.64 $hr^{-1}$ for first 75 min. |
| | 1.27 $hr^{-1}$ 75 min. to 111 min. |

Example 18

Catalyst D-1 from Example 17 was regenerated by calcination in air at 535°C for 6 hours and then cooled to 364°C in a current of $N_2$. A mixture of methanol and water (1:1 v/v) was fed to the reactor for 50 min. The total volume of methanol-water mixture fed to the reactor was 122 $cm^3$ (which contained 48.3 g of methanol). The reaction was carried out for 50 min. and 80.25 g of liquid product was collected containing 78 g of $H_2O$ and 2.25 of organic liquid.

| | |
|---|---|
| % $(CH_2)$ converted to organic liquid | 10.7 |

WHSV 3.4 $hr^{-1}$ (total feed)

1.41 $hr^{-1}$ (based on methanol feed)

The reduced proportion of aromatics and the increase in the proportion of $C_2H_4$ and $C_3H_6$ in the product of Example 18 compared to that of Example 17 are largely the result of the addition of water to the feed of Example 18.

| CONVERSION CONDITIONS | | | | | |
|---|---|---|---|---|---|
| Example No. | Catalyst | Pressure (MPa) | Temperature (°C) | Flow Rate ($cm^3$/hr.) | Sample Time (min.) |
| 1A | A-1 | 0.55 | 424 | 80 | 6 |
| 1B | A-1 | 0.55 | 424 | 80 | 12 |
| 1C | A-1 | 0.55 | 424 | 80 | 23 |
| 1D | A-1 | 0.55 | 424 | 80 | 36 |
| 1E | A-1 | 0.55 | 424 | 80 | 43 |
| 2 | A-1 | 0.55 | 430 | 76 | 85 |
| 3 | A-1 | 0.55 | 432 | 90 | 43 |
| 4 | A-1 | 0.55 | 378 | 90 | 28 |
| 5A | A-1 | atmos. | 317 | 90 | 13 |
| 5B | A-1 | atmos. | 345 | 90 | 30 |
| 5C | A-1 | atmos. | 364 | 90 | 75 |
| 6 | B-1 | atmos. | 400-445 | 53 | 76 |
| 7 | B-1 | atmos. | 363-393 | 47 | 32 |
| 8 | B-1 | atmos. | 393-433 | 89 | 73 |
| 9 | B-1 | atmos. | 454 | 62 | 24 |
| 10A | B-1 | atmos. | 468 | 60 | 58 |
| 10B | B-1 | atmos. | 468-446 | 60 | 98 |
| 10C | B-1 | atmos. | 446-436 | 60 | 106 |
| 11A | B-2 | atmos. | 371 | 20 | 4 |
| 11B | B-2 | atmos. | 373 | 20 | 10 |
| 11C | B-2 | atmos. | 403 | 36 | 28 |

| CONVERSION CONDITIONS | | | | | |
|---|---|---|---|---|---|
| Example No. | Catalyst | Pressure (MPa) | Temperature (°C) | Flow Rate ($cm^3$/hr.) | Sample Time (min.) |
| 11D | B-2 | atmos. | 403 | 36 | 60 |
| 11E | B-2 | atmos. | 399 | 36 | 90 |
| 12A | B-3 | 1 | 388 | 26 | 32 |
| 12B | B-3 | 1 | 391 | 26 | 47 |
| 12C | B-3 | 1 | 391 | 26 | 65 |
| 12D | B-3 | 1 | 391 | 26 | 163 |
| 13A | B-3 | 1 | 384 | 27 | 35 |
| 13B | B-3 | 1 | 395 | 27 | 100 |
| 13C | B-3 | 1 | 393 | 27 | 132 |
| 13D | B-3 | 1 | 413 | 27 | 173 |
| 14A | B-4 | 1 | 377 | 30 | 13 |
| 14B | B-4 | 1 | 393 | 30 | 18 |
| 14C | B-4 | 1 | 412 | 30 | 46 |
| 14D | B-4 | 1 | 414 | 30 | 65 |
| 15A | C-1 | atmos. | 462 | 31 | 4 |
| 15B | C-1 | atmos. | 416 | 31 | 13 |
| 15C | C-1 | atmos. | 419 | 31 | 45 |
| 15D | C-1 | atmos. | 400 | 31 | 72 |
| 16A | C-1 | atmos. | 393 | 71 | 3 |
| 16B | C-1 | atmos. | 411 | 71 | 33 |
| 16C | C-1 | atmos. | 399 | 71 | 52 |

| CONVERSION CONDITIONS | | | | | |
|---|---|---|---|---|---|
| Example No. | Catalyst | Pressure (MPa) | Temperature (°C) | Flow Rate ($cm^3$/hr.) | Sample Time (min.) |
| 16D | C-1 | atmos. | 401 | 71 | 77 |
| 17A | D-1 | atmos. | 389 | 28 | 11 |
| 17B | D-1 | atmos. | 412 | 28 | 31 |
| 17C | D-1 | atmos. | 419 | 28 | 72 |
| 17D | D-1 | atmos. | 478 | 80 | 87 |
| 17E | D-1 | atmos. | 449 | 80 | 108 |
| 18A | D-1 | atmos. | 460 | 146 | 7 |
| 18B | D-1 | atmos. | 449 | 146 | 22 |
| 18C | D-1 | atmos. | 443 | 146 | 45 |
| 18D | D-1 | atmos. | 424 | 146 | 50 |

| Example No. | PRODUCT DISTRIBUTION - WT. % | | | | | | | | | | | | | |
|---|---|---|---|---|---|---|---|---|---|---|---|---|---|---|
| | i-$C_4H_{10}$ | n-$C_4H_{10}$ | 1-$C_4$-$H_8$ | i-$C_4H_8$ | trans 2-$C_4H_8$ | cis 2-$C_4H_8$ | $C_3H_8$ | $C_3H_6$ | $C_2H_6$ | $C_2H_4$ | $CH_4$ | CO | $CO_2$ | $H_2$ |
| 1A | 29.0 | 4.77 | 1.03 | 4.37 | 2.18 | 1.38 | 20.57 | 13.60 | 1.44 | 13.92 | 4.85 | 1.43 | 1.12 | 0.34 |
| 1B | 31.03 | 5.30 | 0.88 | 3.95 | 2.05 | 1.32 | 22.51 | 10.85 | 1.64 | 12.06 | 5.01 | 1.68 | 1.38 | 0.36 |
| 1C | 24.14 | 4.05 | 1.34 | 5.43 | 2.68 | 1.73 | 20.87 | 15.72 | 1.29 | 15.64 | 4.54 | 1.21 | 0.96 | 0.40 |
| 1D | 28.16 | 5.20 | 1.17 | 4.52 | 2.36 | 1.56 | 19.98 | 14.22 | 1.56 | 13.25 | 4.55 | 1.68 | 1.40 | 0.39 |
| 1E | 25.05 | 3.97 | 1.05 | 4.22 | 2.01 | 1.29 | 19.84 | 15.98 | 1.67 | 15.45 | 5.26 | 2.10 | 1.48 | 0.63 |
| 2 | 28.42 | 3.61 | 0.61 | 1.52 | 1.21 | 0.76 | 19.18 | 12.05 | 1.62 | 22.67 | 5.33 | 1.67 | 1.19 | 0.16 |
| 3 | 28.89 | 4.93 | 1.29 | 3.08 | 2.83 | 1.80 | 16.77 | 17.93 | 0.69 | 13.50 | 2.79 | 4.88 | 0.61 | 0.02 |
| 4 | 29.18 | 3.95 | 0.99 | 2.21 | 1.76 | 1.25 | 14.39 | 13.71 | 5.23 | 23.75 | 2.36 | 0.66 | 0.50 | 0.05 |
| 5A | 27.74 | 4.40 | 1.03 | 3.36 | 2.11 | 1.66 | 11.14 | 15.18 | 3.52 | 27.76 | 1.46 | 0.00 | 0.63 | 0.00 |
| 5B | 17.39 | 2.20 | 1.31 | 2.43 | 3.06 | 1.98 | 6.84 | 35.36 | 0.21 | 27.16 | 1.58 | 0.04 | 0.38 | 0.05 |
| 5C | 20.34 | 2.33 | 1.31 | 3.62 | 3.07 | 2.01 | 7.18 | 34.42 | 0.58 | 23.21 | 1.39 | 0.01 | 0.44 | 0.07 |
| 6 | 39.0 | 7.7 | 1.26 | 10.28 | 2.34 | 3.75 | 15.67 | 7.34 | 0.61 | 8.26 | 2.27 | 0.55 | 0.31 | 0.21 |
| 7 | 19.53 | 2.06 | 2.45 | 13.30 | 4.70 | 3.52 | 5.52 | 28.55 | 0.44 | 13.99 | 2.08 | 1.14 | 2.6 | 0.12 |

| Example No. | PRODUCT DISTRIBUTION - WT. % | | | | | | | | | | | | | |
|---|---|---|---|---|---|---|---|---|---|---|---|---|---|---|
| | $i\text{-}C_4H_{10}$ | $n\text{-}C_4H_{10}$ | $1\text{-}C_4\text{-}H_8$ | $i\text{-}C_4H_8$ | trans $2\text{-}C_4H_8$ | cis $2\text{-}C_4H_8$ | $C_3H_8$ | $C_3H_6$ | $C_2H_6$ | $C_2H_4$ | $CH_4$ | CO | $CO_2$ | $H_2$ |
| 8 | 15.78 | 1.99 | 2.66 | 13.2 | 3.25 | 4.8 | 5.84 | 34.79 | 0.26 | 12.80 | 2.24 | 1.53 | 0.68 | 0.18 |
| 9 | 33.46 | 6.39 | 1.18 | 7.49 | 2.01 | 1.60 | 18.61 | 11.43 | 1.05 | 7.43 | 6.64 | 1.50 | 0.74 | 0.47 |
| 10A | 32.65 | 4.93 | 0.89 | 5.54 | 1.55 | 1.03 | 20.04 | 11.22 | 1.65 | 9.42 | 8.33 | 1.94 | 0.37 | 0.44 |
| 10B | 34.68 | 4.56 | 0.84 | 6.11 | 1.46 | 1.02 | 18.87 | 9.36 | 1.18 | 10.01 | 9.51 | 1.76 | 0.42 | 0.21 |
| 10C | 35.15 | 5.08 | 0.91 | 6.71 | 1.75 | 1.15 | 18.29 | 9.60 | 1.06 | 10.61 | 7.56 | 1.42 | 0.39 | 0.31 |
| 11A | 49.29 | 8.21 | 1.70 | 9.06 | 4.39 | 2.83 | 14.02 | 2.12 | 0.38 | 4.18 | 1.90 | 0.00 | 1.22 | 0.68 |
| 11B | 42.56 | 5.69 | 1.10 | 6.05 | 2.20 | 1.51 | 16.20 | 11.34 | 0.74 | 7.77 | 2.36 | 0.00 | 1.86 | 0.63 |
| 11C | 37.36 | 5.45 | 1.32 | 7.50 | 2.76 | 1.84 | 16.03 | 14.62 | 0.63 | 8.23 | 3.38 | 0.00 | 0.62 | 0.24 |
| 11D | 34.89 | 3.80 | 0.99 | 6.06 | 2.54 | 1.13 | 15.95 | 14.80 | 0.83 | 11.91 | 4.95 | 1.34 | 0.55 | 0.27 |
| 11E | 32.90 | 3.42 | 1.01 | 5.75 | 1.58 | 1.01 | 16.03 | 15.31 | 1.39 | 14.01 | 5.58 | 1.51 | 0.34 | 0.17 |
| 12A | 36.46 | 3.49 | 1.48 | 7.55 | 2.56 | 1.75 | 11.13 | 16.89 | 0.94 | 11.67 | 3.39 | 1.75 | 0.74 | 0.18 |
| 12B | 34.04 | 2.91 | 1.40 | 7.16 | 2.25 | 1.54 | 11.15 | 18.85 | 0.68 | 12.92 | 4.45 | 1.40 | 0.99 | 0.25 |
| 12C | 33.81 | 3.68 | 1.84 | 9.08 | 3.69 | 2.50 | 10.14 | 19.45 | 0.63 | 9.94 | 3.54 | 0.74 | 0.83 | 0.13 |

| Example No. | PRODUCT DISTRIBUTION - WT. % | | | | | | | | | | | | | |
|---|---|---|---|---|---|---|---|---|---|---|---|---|---|---|
| | $i-C_4H_{10}$ | $n-C_4H_{10}$ | $1-C_4-H_8$ | $i-C_4H_8$ | trans $2-C_4H_8$ | cis $2-C_4H_8$ | $C_3H_8$ | $C_3H_6$ | $C_2H_6$ | $C_2H_4$ | $CH_4$ | CO | $CO_2$ | $H_2$ |
| 12D | 31.48 | 2.66 | 1.43 | 7.42 | 2.43 | 1.57 | 10.20 | 20.65 | 0.69 | 14.84 | 4.73 | 0.64 | 1.01 | 0.26 |
| 13A | 32.95 | 3.33 | 1.45 | 7.71 | 3.21 | 2.09 | 9.09 | 18.80 | 0.69 | 10.20 | 1.74 | 4.10 | 3.41 | 1.22 |
| 13B | 26.57 | 2.19 | 1.69 | 8.60 | 2.82 | 1.83 | 8.53 | 26.11 | 0.53 | 15.43 | 3.46 | 1.06 | 1.00 | 0.19 |
| 13C | 26.44 | 2.09 | 1.73 | 8.37 | 2.60 | 1.73 | 8.16 | 25.96 | 0.54 | 16.23 | 3.54 | 1.08 | 1.25 | 0.28 |
| 13D | 26.61 | 2.37 | 1.86 | 8.99 | 2.85 | 1.86 | 8.75 | 25.26 | 0.61 | 14.27 | 4.00 | 1.21 | 1.12 | 0.25 |
| 14A | 47.13 | 8.74 | 0.26 | 1.79 | 1.02 | 0.51 | 25.11 | 4.12 | 1.03 | 2.43 | 3.10 | 1.66 | 2.81 | 0.30 |
| 14B | 42.90 | 5.35 | 0.41 | 2.31 | 0.68 | 0.54 | 27.11 | 7.23 | 1.38 | 5.16 | 3.57 | 1.70 | 1.28 | 0.38 |
| 14C | 37.42 | 6.19 | 0.53 | 2.92 | 1.06 | 0.66 | 29.96 | 9.07 | 2.06 | 5.11 | 3.07 | 0.93 | 0.73 | 0.27 |
| 14D | 37.33 | 6.56 | 0.52 | 3.10 | 1.03 | 0.65 | 29.74 | 9.11 | 2.42 | 5.30 | 2.73 | 0.84 | 0.51 | 0.16 |
| 15A | 23.47 | 6.16 | 2.12 | 9.63 | 4.39 | 2.97 | 14.91 | 22.20 | 1.37 | 8.85 | 2.71 | 0.71 | 0.00 | 0.51 |
| 15B | 29.48 | 6.24 | 1.54 | 6.87 | 2.80 | 1.96 | 18.95 | 18.61 | 1.20 | 7.71 | 2.64 | 0.70 | 0.77 | 0.52 |
| 15C | 29.90 | 4.48 | 1.15 | 5.20 | 1.59 | 1.01 | 20.98 | 19.81 | 1.08 | 9.60 | 3.34 | 0.79 | 0.57 | 0.50 |
| 15D | 33.83 | 4.43 | 1.00 | 5.13 | 1.28 | 0.86 | 21.52 | 16.58 | 1.30 | 9.27 | 3.30 | 0.71 | 0.34 | 0.45 |

| PRODUCT DISTRIBUTION - WT. % | | | | | | | | | | | | | | |
|---|---|---|---|---|---|---|---|---|---|---|---|---|---|---|
| Example No. | $i-C_4H_{10}$ | $n-C_4H_{10}$ | $1-C_4-H_8$ | $i-C_4H_8$ | trans $2-C_4H_8$ | cis $2-C_4H_8$ | $C_3H_8$ | $C_3H_6$ | $C_2H_6$ | $C_2H_4$ | $CH_4$ | CO | $CO_2$ | $H_2$ |
| 16A | 28.77 | 4.07 | 2.09 | 10.57 | 4.79 | 3.20 | 8.69 | 23.05 | 0.20 | 10.26 | 0.70 | 0.00 | 3.48 | 0.12 |
| 16B | 19.23 | 2.40 | 2.84 | 14.70 | 5.80 | 4.00 | 5.98 | 31.24 | 0.21 | 11.99 | 1.07 | 0.26 | 0.20 | 0.09 |
| 16C | 19.76 | 2.17 | 2.74 | 13.46 | 5.36 | 3.66 | 5.65 | 31.17 | 0.21 | 14.24 | 1.08 | 0.33 | 0.10 | 0.07 |
| 16D | 19.07 | 2.04 | 2.63 | 13.15 | 5.00 | 3.42 | 5.68 | 32.94 | 0.21 | 14.14 | 1.20 | 0.33 | 0.10 | 0.08 |
| 17A | 43.79 | 8.41 | 0.50 | 2.32 | 1.16 | 0.66 | 20.45 | 5.84 | 1.69 | 3.81 | 2.42 | 6.88 | 0.52 | 1.55 |
| 17B | 40.02 | 6.81 | 0.80 | 4.02 | 1.61 | 1.07 | 22.87 | 10.97 | 1.01 | 5.17 | 3.56 | 0.87 | 0.84 | 0.36 |
| 17C | 39.41 | 6.34 | 0.80 | 4.52 | 1.60 | 1.06 | 23.31 | 11.08 | 1.50 | 5.72 | 3.76 | 0.60 | 0.00 | 0.29 |
| 17D | 41.17 | 8.13 | 1.16 | 7.07 | 2.57 | 1.67 | 18.09 | 9.26 | 1.45 | 4.89 | 3.64 | 0.45 | 0.20 | 0.25 |
| 17E | 35.44 | 8.39 | 1.44 | 8.49 | 3.26 | 2.09 | 18.47 | 9.70 | 1.26 | 5.94 | 4.59 | 0.46 | 0.31 | 0.18 |
| 18A | 24.77 | 4.13 | 2.57 | 12.34 | 5.40 | 3.73 | 9.60 | 23.91 | 0.41 | 9.90 | 2.17 | 0.58 | 0.40 | 0.10 |
| 18B | 18.29 | 2.84 | 2.60 | 12.46 | 5.07 | 3.42 | 8.28 | 28.65 | 0.59 | 13.69 | 2.78 | 0.75 | 0.43 | 0.15 |
| 18C | 13.03 | 1.39 | 2.95 | 11.65 | 5.22 | 3.61 | 4.10 | 42.48 | 0.43 | 12.85 | 1.68 | 0.33 | 0.21 | 0.06 |
| 18D | 24.14 | 2.62 | 2.66 | 12.92 | 4.81 | 3.17 | 8.66 | 34.38 | 0.47 | 2.41 | 2.50 | 0.82 | 0.30 | 0.14 |

CLAIMS

1. A process for preparing a hydrocarbon product rich in iso-$C_4$ compounds, which comprises contacting methanol or dimethyl ether, or a mixture thereof, with a catalyst comprising a crystalline silica having molecular sieve properties and a substantially uniform pore diameter, at 300 to 550°C.

2. A process for preparing a hydrocarbon product rich in iso-$C_4$ compounds, which comprises contacting methanol or dimethyl ether, or a mixture thereof, with a catalyst comprising a crystalline solid having molecular sieve properties and a substantially uniform pore diameter, the solid comprising silica and impregnated with one or more of the oxides of thorium, zirconium and titanium, at 300 to 550°C.

3. A process according to claim 2, in which the crystalline solid is crystalline silica or crystalline aluminosilicate.

4. A process according to claim 2 or claim 3, in which the catalyst contains up to 50% by weight of the oxide or oxides.

5. A catalyst composition which comprises crystalline silica having molecular sieve properties and a substantially uniform pore diameter, and impregnated with from 0.5 to 50% by weight of one or more of the oxides of thorium, zirconium and titanium.

6. A process according to any of claims 2 to 4 or a composition according to claim 4, in which the catalyst contains 0.5 to 30, preferably 2 to 15, % by weight of the oxide or oxides.

7. A process or composition according to any of claims 2 to 6, in which the oxide is thorium oxide.

8. A process or composition according to any preceding claim, in which the catalyst is crystalline silica substantially free of alumina, e.g. silicalite.

9. A process or composition according to any

preceding claim, in which the catalyst has a pore diameter of 5 to 10 Angstrom units.

10. A process according to any preceding claim, which is conducted at 330 to 455°C.

11. A process according to any preceding claim, in which the methanol and/or dimethyl ether is in admixture with water, preferably in an amount of up to 80% by weight.

12. A process according to any preceding claim, in which methanol is used, in a 60:40 to 30:70 w/w mixture with water.

13. A process according to claim 11, in which an equilibrium mixture of methanol, dimethyl ether and water is used.

European Patent Office

# EUROPEAN SEARCH REPORT

0100604
Application number

EP 83303852.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US - A - 4 320 241 (FRANKIEWICZ) * Claims 1,3,7,9; column 4, lines 64-66; column 5, lines 59-63 * | 1,8-10, 11 | C 07 C 1/20<br>C 07 C 9/12<br>C 07 C 11/09<br>B 01 J 29/04<br>//B 01 J 29/28 |
| X | DE - A1 - 2 909 927 (BASF) * Claim 1; example; page 3, line 22 * | 1,10,11 | |
| X | US - A - 4 013 732 (CHANG et al.) * Claims 1-5; table 1 * | 2-4,9, 10 | |
| A | GB - A - 1 589 857 (MOBIL OIL) * Page 2, lines 7-28; example 2 * | 1,9,12 | TECHNICAL FIELDS SEARCHED (Int. Cl. 3) |
| A | DE - A1 - 2 935 863 (MOBIL OIL) * Claims; page 10, lines 1,6-9; page 11, line 2; page 23, lines 17-26; table III * | 2,4,6, 7,9,11, 13 | C 07 C 1/00<br>C 07 C 9/00<br>C 07 C 11/00<br>B 01 J |
| D,A | US - A - 4 061 724 (GROSE et al.) | 1 | |
| A | US - A - 3 329 480 (YOUNG et al.) | 5 | |

The present search report has been drawn up for all claims

| Place of search | Date of completion of the search | Examiner |
|---|---|---|
| VIENNA | 05-10-1983 | KÖRBER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document